# EUROPEAN PATENT APPLICATION

(11) **EP 4 393 450 A1**
(43) Date of publication of application: **03.07.2024**
(21) Application number: 22217052.4
(22) Date of filing: 29.12.2022
(51) Int. Cl.: A61F 2/02, A61B 17/12, D04C 1/06

(54) **MANUFACTURING METHOD FOR A MEDICAL IMPLANTABLE DEVICE AND A MEDICAL IMPLANTABLE DEVICE AND BASE BODY FOR SUCH IMPLANTABLE DEVICE**

(71) Applicant: OCCLUTECH GmbH, 07745 Jena (DE)
(72) Inventor: Soyutemiz, Secil, Sutton (GB); Karaman, Ugur, Sutton (GB); Çavusolu, Olcay, Istanbul (TR); Isilkli, Cansel, Istanbul (TR)
(74) Representative: KIPA AB

(57) **Abstract**

A method for forming a braided tubular base body (300) for a medical implantable device (400) is disclosed. The method comprises providing a braiding head (102) having form elements (100), and a plurality of bobbins (202, 204). The method comprises providing a plurality of first-stage wires (104) and forming a plurality of first-stage apices. The method comprises providing a plurality of second-stage wires (106) and forming a plurality of second-stage apices. Each second-stage apex is located between adjacent first-stage apices. The method further comprises braiding a tubular base body (300) for a medical implant (400) from the first and second-stage wires (104, 106).

## Description

### TECHNICAL FIELD

The present invention relates to the manufacturing of medical implants, in particular involving braiding of wires or strands and methods thereof, as well as base bodies manufactured of such braiding for forming medical implants including in particular shunting devices such as septal shunting devices, wherein such implants are of a braided mesh of strands.

### BACKGROUND

Various braided medical devices are used for treating various conditions in a patient. In certain circumstances it may be necessary to use such devices for occlusion of a patient's lumen, vessel, chamber, channel, hole, or cavity. Braided devices may also be used for shunting. When delivering or implanting such devices into the patient's body it is critical that the braided device is sufficiently flexible for safe delivery by a delivery device such as a catheter to a target site in the patient. The ease of operation by which the medical device can be delivered is crucial from several aspects such as requirements to comply with time limits for a quick treatment or overall safe positioning or maneuvering of the device at the target site.

The braided medical devices often comprise one or more wires or strands used to braid a base body that is then used to form the medical device, often by heat-setting. The ends of the wires or strands are commonly fixed together to avoid unraveling of the braid, e.g. in fixtures, bundles or securing means which often are protruding from the medical device.

Some braiding machines can be configured and set up to braid base bodies that may have open and/or closed ends. Different braiding techniques and set ups have been used to braid sturdy and flexible base bodies for medical devices.

For example, in US6468303B1, or WO99/12478A1 medical devices are disclosed made from tubular braiding and have securing means, such as a clamp, at each respective opposite end of the device. The securing means, however, in particular in the left heart, may increase a risk for blood clotting or other complications, since e.g. the distal clamp protrude from the device. This which may in particular be an issue in the left heart leading to high risk of undesired complications like stroke caused by blood clots, or punctures of cardiac tissue during deployment of the device. In international patent application publication WO2005/020822A1 an occlusion device is disclosed and in international patent application publication WO2016/038115A1 a shunt device is disclosed, wherein both disclose devices that are made from braided base bodies. These latter medical devices have one securing means, such as a clamp at only the proximal end. In another example In US9877726B2 an occlusion device is disclosed having one clamp at one end, and a braided opening at the other end that is tied together with a suture thread to restrain the opening into forming a closed smooth surface. However, there is a need for further improved braided medical devices, base bodies for such devices and manufacturing methods for such base bodies and/or devices.

### SUMMARY

Accordingly, embodiments of the present invention preferably seek to mitigate, alleviate or eliminate one or more deficiencies, disadvantages or issues in the art, such as the above-identified, singly or in any combination by providing devices, assemblies, kits, systems and methods according to the appended patent claims. The present invention is defined by the appended claims only, in particular by the scope of appended independent claims. References to "embodiments" throughout the description which are not under the scope of the appended claims merely represent possible exemplary executions and are therefore not part of the present invention. Particular technical effects and advantages of specific features or steps of the disclosure are mentioned below.

In one aspect of the disclosure, there is provided a method of forming a braided tubular base body for a medical implant. The method comprises in examples providing a plurality of form elements at one end of a braiding head. The braiding head commonly has a central axis. The form elements are disposed of a first arrangement around said central axis. The method comprises in examples providing a plurality of pairs of first-stage wires. A plurality of groups of first-stage apices is formed by hooking each pair of first-stage wires over a first and a second form element. Further, a plurality of pairs of second-stage wires is provided. A plurality of groups of second-stage apices each located between adjacent first-stage apices is formed by hooking each pair of second-stage wires over the first and second form elements enveloping the first-stage apices. Subsequently, the first and second-stage wires are braided to the said tubular base body.

The enveloping of the first-stage apices may improve the durability of the tubular braid. The present braiding may thus improve patient safety by the intertwining of groups of first and second-stage apices.

The tubular base body manufactured according to a method of the disclosure or having the structural features disclosed herein is provided with flexibility which facilitates handling of the tubular base body. The tubular base body is easy to form into a medical device. When braiding the tubular base body, loose wire ends end up on the same side. With all free wire ends at one side, when forming a medical device, a medical device may be formed with only one closure element at one end for securing any loose ends of strands of the braid and preventing fraying. In such cases, patient safety can be improved by limiting the number of protruding elements from the medical device. Which can reduce the risk of blood clotting in connection with the medical implantable device formed by the braided base body.

This is specifically beneficial when the medical device is placed in a septal wall, or in the interatrial septum. The formation of the enveloping apices may provide the tubular base body with rigidity and flexibility to be able to form a variety of medical devices such as an occlusion device or a shunting device.

By using more than one wire or strand to form a group of apices, an opening can be formed by the braiding setup that can hold open by itself, and that may facilitate in makings of shunting devices or occlusion devices.

In some examples, the method comprises providing a plurality of bobbins. The bobbins may be arranged in second and third concentric arrangements outwards from the central axis of the braiding head. The group of first-stage apices may be formed by a first and a second first-stage wire. Ends of the first first-stage wires may be led out from the first apex to a first bobbin of the second arrangement and a first bobbin of the third arrangement respectively. The first bobbins of the second and third arrangements may be concentrically aligned. Ends of the second first-stage wires may be led out from the apex to a second bobbin of the second arrangement and a second bobbin of the third arrangement respectively. The second bobbins of the second and third arrangements may be concentrically aligned. The second bobbin of the second arrangement may be adjacent to the first bobbin of the second arrangement. The second bobbin of the third arrangement may be adjacent to the first bobbin of the third arrangement.

In some examples, each pair of first and second bobbins may be adjacent to a pair of third and fourth bobbins without any first-stage wires connected thereto.

In some examples, the group of second-stage apices may be formed by a first and a second-stage wire. One end of the first second-stage wire may be led out to a third bobbin of the second arrangement. The third bobbin may be adjacent to a second bobbin of the second arrangement. The other end of the first second-stage wire may be led out to a fourth bobbin of the third arrangement. One end of the second-stage wire may be led out to a third bobbin of the third arrangement. The third bobbin of the third arrangement may be adjacent to the second bobbin of the third arrangement. The other end of the second-stage wire may be led out to a fourth bobbin of the second arrangement. The fourth bobbin may be adjacent to a first bobbin of another group of first-stage apices. Such a configuration may improve the flexibility of the braided tubular base body.

In some examples, the bobbins may be provided in a ratio of form elements to bobbins being 1:4. Such a set-up of the braiding machine may facilitate the set-up of the wires. Such a setup may improve the flexibility and durability of the braided tubular base body. In some examples the bobbins may be provided in a ratio of form elements to bobbins being 1:8.

In some examples, there may be provided forty bobbins in the second arrangement. There may be provided forty bobbins in the third arrangement. The second and third arrangement of bobbins may be consecutively arranged. In some examples, the step of providing a plurality of form elements may comprise providing ten form elements.

Different numbers of form elements and bobbins may be advantageous to provide a variety of sizes of braided tubular base bodies.

In another aspect, there is provided a method for forming a medical implantable device from a braided tubular base body. The method comprises forming a tubular base body according to any one of the examples above. The method further comprises heat treating the tubular base body to form the medical implantable device from the braided tubular base body.

In another aspect, there is provided method for forming a medical implantable device. The method comprises braiding a tubular base body preferably according to any one of the above examples. The tubular body comprises a plurality of groups of first-stage apices. The base body comprises a plurality of groups of second-stage apices each second-stage apex located between adjacent first-stage apices. The method further comprises providing a mold structure. The method comprises inserting the tubular braid into the mold structure. The method comprises inserting a peg through the tubular braid in the mold structure for forming a channel through the medical implantable device. The method further comprises treating the mold structure with a heat setting to form the medical implantable device.

In some examples, the peg may be inserted at the center of the mold. The peg may have a conical tip providing for an easier, less time-consuming, and costly manufacturing method.

In some examples, the method may comprise fixating at least a portion of the ends of the wires together.

In some examples, the method may comprise fixating said wires at a distance offset a central axis of the medical implantable device.

In another aspect, there is provided a tubular base body for a medical implant, preferably a shunting or occlusion device. The base body has a plurality of first-stage wires, and a plurality of second-stage wires. The base body includes a plurality of groups of first-stage apices. The base body further includes a plurality of groups of second-stage apices each located between adjacent first-stage apices.

In another aspect, there is provided an implantable medical device. Preferably made from a tubular base body of the above aspect. The medical implantable device includes a plurality of first-stage wires, and a plurality of second-stage wires. The medical implantable device includes a plurality of groups of first-stage apices. The medical implantable device further includes a plurality of groups of second-stage apices each located between two adjacent first-stage apices.

In some examples, the medical implantable device may comprise a channel through the medical implantable device.

The opening mentioned above can thus advantageously be provided at an end of the base body without any elements extending beyond the end. Medical devices are thus providable with an advantageous opening at an end thereof, without protruding elements (like bundles of wire, securing means, or the like). Such openings provided from base bodies manufactured as described herein advantageously provide long-term openings at an end (or a channel through the) medical device thus formed from the base body (preferably by using a mold and heat setting process). The opening does thus advantageously not grow over or cause endothelization over the opening when implanted. Moreover, the base body thus formed provides a sturdy guide at the perimeter of the opening at its end for a forming element inserted into the braiding of the base body, such as a peg, e.g. when inserted into a mold to form a medical device. Thus, the obtained tubular braided base body provides advantageously facilitating of manufacture of a medical implantable device having an opening at an end thereof and preferably having a through channel (such as a shunting device or occlusion device/occluder) in a secure and reliable manner.

### BRIEF DESCRIPTION OF DRAWINGS

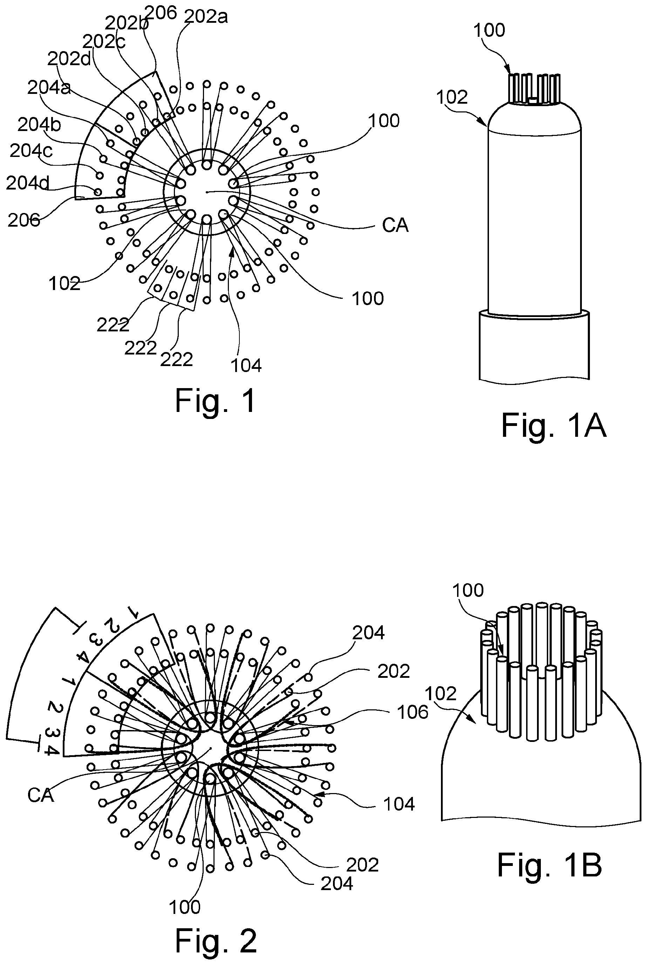

These and other aspects, features and advantages of which embodiments of the invention are capable of will be apparent and elucidated from the following description of embodiments of the present invention, reference being made to the accompanying drawings, in which
Figure 1A is a lateral view of an example of a braiding head 102 with a cylindrical body section, a cupped top section and a plurality of form elements 100;
Figure 1B is an elevation view of a detail of the braiding head 102 top;
Figure 1 is a schematic top view of a braiding machine comprising a braiding head having a plurality of form elements, and a plurality of bobbins and a plurality of wires hooked over the form elements and their ends connected to the bobbins;
Figure 2 is a schematic top view of a braiding machine comprising a braiding head having a plurality of form elements, a plurality of bobbins and a plurality of wires hooked over the form elements and their ends connected to the bobbins;
Figure 3A is a schematic side view of a braided tubular base body, the braided tubular base body having a proximal end, and a distal end, wherein the ends of the wires are at the proximal end, and where the ends of the wires are fixated together;
Figure 3B is a schematic view of a distal end of an example of a tubular base body, having an oval shape opening at an end thereof;
Figures 3C and 3D are each a schematic side view of an example of a distal end of a tubular base body, having an opening with a smaller diameter than the diameter of the braided tubular base body;
Figure 3E is an elevation view of an example of a braided tubular base body manufactured in accordance with methods disclosed herein;
Figure 4A is a schematic side view of a mold structure for forming a medical device;
Figure 4B is a schematic side view of a braided tubular base body partly inserted into a mold structure;
Figure 4C is a schematic view of a peg being inserted into a mold structure for forming a channel through a medical device;
Figure 4D is a schematic top view of a braided tubular base body inserted into a mold structure, wherein ends of the braided base body extend at the top side;
Figure 4E is a schematic top view of a mold structure, having a braided tubular base body therein, and a peg through the mold structure for forming a central channel through the medical device, the ends of the wires are extending from the mold structure at a distance offset from the central channel;
Figures 5, 6A, and 6B are flow charts illustrating examples of some methods of the disclosure;
Figures 7A and 7B are schematic illustrations of an example of a medical implantable device 400, in the example with a through-channel made of a braided tubular base body 300.

### DETAILED DESCRIPTION

Specific embodiments of the disclosure will now be described with reference to the accompanying drawings. This invention may, however, be embodied in many different forms and should not be construed as limited to the embodiments set forth herein; rather, these embodiments are provided so that this disclosure will be thorough and complete, and will fully convey the scope of the invention to those skilled in the art. The terminology used in the detailed description of the embodiments illustrated in the accompanying drawings is not intended to be limiting to the invention. In the drawings, like numbers refer to like elements.

The following description focuses on an embodiment of the present invention applicable to methods and devices for forming a medical implantable device, and in particular to a method for braiding a tubular base body and a tubular base body for forming into a medical implantable device.

Many medical implantable devices are made from braided base bodies. The braided base bodies of the disclosure are braided using commonly used 3D braiding machines comprising a braiding head having form elements, and bobbins. The form elements are often arranged around a central part of the braiding head. The bobbins are in turn arranged in a different plane in a direction outward from the center of the braiding head, and outwards from the form elements.

These common braiding machines can form open-ended braided base bodies, or closed-ended base bodies, depending on the use of the braided base body.

When the braiding machine has been set up with wires in accordance with the present disclosure, the machine moves the bobbins in a pattern around the axis of the braiding head to braid the attached wires or threads into a braiding.

An example of a braiding head 102 without loaded wires or strands is shown in Figs. 1A and 1B.

Turning now to Figure 1, a set-up of wires on a braiding head 102 is shown. The example shows a schematic top view of a braiding head 102 shown in Figs. 1A and 1B.

The braiding head 102 may be of circular cross-section as shown in the exemplary figures, and in such embodiments having a central axis CA at the center of the braiding head 102. In other embodiments (not shown) the braiding head may have a non-circular cross-sectional shape, i.e. a cylindrical base resulting in a circular cross section of the corresponding braiding made on the braiding head. The braiding head 102 has a longitudinal extension in the direction of the central axis CA. Arranged outwards from the central axis, on the top of the braiding head 102 is a plurality of form elements 100. The form elements are arranged in a first arrangement outwards from the central axis CA. The first arrangement may have a circular shape, an oval shape, or any shape suitable for the desired medical device to be formed. The form elements are arranged within the periphery of the braiding head 102. Each form element 100 may be arranged having the same distance from the central axis CA. However, changing the distance of at least one of the form elements 100 may alter the end of the braided tubular base body. This may be advantageous in order to facilitate the manufacturing of different sizes of medical implantable devices. It may advantageously facilitate making different types of medical implantable devices.

In some examples, the braiding head 102 comprises ten form elements 100, as shown in Figs. 1A, 1 and 2. However, this is merely an example of a specific number of form elements 100. For instance, the braiding head is shown in Fig., 1B has more form elements 100.

Outwards, and in a second plane below the top of the braiding head 102, there is illustrated a second arrangement 202 comprising a plurality of bobbins. The second plane is in the longitudinal direction of the braiding head 102. Outwards from the second arrangement of bobbins 202, there is shown a third arrangement 204 comprising a plurality of bobbins. The bobbins of the second arrangement 202, and the third arrangement 204 may be consecutively arranged outwards from the central axis CA. The bobbins of the second and third arrangements are preferably arranged outside the periphery of the braiding head. The bobbins of the first and second arrangements may shift place with each other in an undulating pattern when circulated around the central axis, as known for e.g. maypole braiding techniques. The braid is then formed from the top of the braiding head downwards. Other supporting elements of the braiding machine may be present (not shown), such as a holder ring movable along the braiding head to assist the braiding being created down along the braiding head when the bobbins are in braiding motion.

The herein described innovative setup of the braiding machine comprises attaching and arranging the wires that are to be used to braid the braiding to the braiding machine. In the shown embodiment, a set-up is shown for a plurality of first-stage wires 104 for forming a first-stage braiding of the tubular base body.

Each form element 100 has two first-stage wires 104 hooked therearound. Each first-stage wire 104 being hooked around the form element 100 forms an apex at the corresponding form element 100. Each form element 100 (numbered in the example of Figs. 1 and 2 with 1 to 10) provides the setup with two first-stage apices. When setting up the first-stage wires, the wire is attached at a first bobbin, hooked, or led around the form element 100, and led out to a second bobbin and connecting it thereto.

For instance, a wire 104 is hooked to form element 100 with number 1. This wire is looped around the form element with the number 1. Its two wire ends are fixed to bobbins 1A and 1B, respectively. Thereby forming the first-stage apex of this particular wire. A second wire 104 is hooked to the same form element 100 with the number 1. This second wire is also looped around the form element with number 1. Its two wire ends are fixed to bobbins 2B and 2A, respectively. Thereby forming the first-stage apex of this particular second wire around the same form element.

This (two wires looped around a form element) is repeated for each form element 100. Thus, a first and a second first-stage wire 104 forms a group of first-stage apices. In a similar manner, the first-stage wire 104 and the second first-stage wire may form a group of first-stage wires. Hence, the set-up of the braiding machine includes providing a plurality of groups of first-stage wires 104 and forming a plurality of groups of first-stage apices with the first-stage wires 104.

The bobbins of the second and third arrangements 202, 204, may be arranged in groups 206. Each group comprises at least one bobbin from the second arrangement 202, and at least one bobbin from the third arrangement 204. A first bobbin 202a of the second arrangement 202 and a first bobbin 204a of the third arrangement 204 may form a pair 222 of bobbins. Each group 206 may comprise at least one pair 222 of bobbins. Each group 206 may comprise two pairs 222 of bobbins. Preferably, each group 206 comprises four pairs 222 of bobbins. For instance, the group includes bobbins A1, 1B (first wire), 2B, 2A (second wire) and then 3A, 3B, 4B, 4A with no wires connected yet. Bobbins 3A, 3B, 4B, 4A will be fixed to other wire ends as shown in the example of Fig. 2 and described below.

In the schematic top view of the braiding machine, each group 206 comprises four bobbins of the second arrangement 202, and four bobbins of the third arrangement 204.

Each group comprises the first bobbin 202a from the second arrangement 202. The group comprises a second bobbin 202b from the second arrangement 202. The group comprises a third bobbin 202c from the second arrangement 202. The group comprises a fourth bobbin 202d from the second arrangement 202.

The group further comprises the first bobbin 204a from the third arrangement 204. The group comprises a second bobbin 204b from the third arrangement 204. The group comprises a third bobbin 204c from the third arrangement 204. The group comprises a fourth bobbin 204d from the third arrangement 204.

As mentioned above, in the shown example of the disclosure, the first end of the first first-stage wire 104 is connected to the first bobbin 202a of the second arrangement 202. Then, the first first-stage wire 104 is wound around the form element 100. The second end of the first first-stage wire 104 is led out from the form element to the first bobbin 204a of the third arrangement 204 and connected thereto. The first apex of a group of first-stage apices is thereby formed.

A second apex is formed by the second first-stage wire 104. The first end of the second first-stage wire 104 is connected to the second bobbin 202b of the second arrangement 202. Then, the second-stage wire is hooked around the same form element 100 as the first first-stage wire 104. The second end of the second first-stage wire is led out to the second bobbin 204b of the third arrangement 204. Thereby forming the second first-stage apex. The first and second apex are overlapping each other around the form element 100.

The first and second bobbins 202a, 202b of the second arrangement 202 are located adjacent one another. The first and second bobbins 204a, 204b of the third arrangement 204 are located adjacent one another.

The third 202c, 204c and fourth 202d, 204d bobbins of the second 202 and third 204 arrangements are preferably left without any first-stage wires 104 connected thereto. The third 202c, 204c and fourth 202d, 204d bobbins of the second 202 and third 204 arrangements are adjacent to the first 202a, 204a and the second 202b, 204b bobbins of the second 202 and third 204 arrangements.

A second group of first-stage apices is formed by hooking a pair of first-stage wires 104 around another form element 100. The ends of the first-stage wired 104 of the second group of first-stage apices are led out to bobbins of a second group 222.

The second group of first-stage apices has the first bobbins of the second and third arrangement adjacent to the fourth bobbins of the second and third arrangement of the first group. This leaves two pairs 222 of bobbins empty between each group of first-stage wires 104 in the setup of the first-stage wires 104.

Now turning to Figure 2 showing a schematic top view of the exemplary braiding machine, in a setup for forming a braided tubular base body comprising a plurality of first-stage apices and a plurality of second-stage apices. Each second-stage apex is formed by a second-stage wire 106.

Each second-stage apex is formed by connecting the first end of a first second-stage wire 106 to the third 202c bobbin of the second arrangement 202. Leading the first second-stage wire 106 around and hooking it over two adjacent form elements 100. The second end of the first second-stage wire 106 is led out to a fourth bobbin 204d of the third arrangement 204. Thereby forming a first second-stage apex. The first- and second-second-stage wires 106 are preferably hooked around the same two form elements 100.

The fourth bobbin 204d is preferably the fourth bobbin 204d of a different group 206 of bobbins than the third bobbin 202c whereto the first end of the first second-stage wire 106 is connected.

The second-stage apex may be formed by hooking the second-stage wire around at least 2 form elements 100. In some examples, the second-stage apex may be formed by hooking the second-stage wire around 3 or 4 form elements 100.

Each second-stage apex is positioned or located between two adjacent groups of first-stage apices. When forming the second-stage apex, the second-stage apex envelops and/or overlap two groups of first-stage apices. In some examples, the second-stage apex may envelope two adjacent groups of first-stage apices. This may provide the tubular base body with rigidity to keep an open end, and to keep the base body flexible to form a medical device.

For instance, in a non-limiting example, a first second-stage wire 106 is looped over the form elements 100 with the number 1 and 2 in the illustrated example. The apex of this first second-stage wire 106 is thus located between the adjacent form elements 100 with the number 1 and 2. The first end of this first second-stage wire 106 is affixed to bobbin 204 with the number 3A. The second end of this first second-stage wire 106 is affixed to bobbin 202 with the number 8A. A second second-stage wire 106 is looped over the form elements 100 with the number 1 and 2 in the illustrated example. The apex of this first second-stage wire 106 is thus also located between the adjacent form elements 100 with the number 1 and 2. The first end of this second second-stage wire 106 is affixed to bobbin 202 with the number 3B. The second end of this first second-stage wire 106 is affixed to bobbin 204 with the number 8B.

This is repeated for further second-stage wires 106 as illustrated in Fig. 2, e.g. around form elements 2 and 3 (apex between 2 and 3), and bobbins 7A, 12A and .7B, 12B, respectively, and so on until all bobbins are loaded with wires.

A second second-stage wire 106 is connected to the third 204c bobbin of the third arrangement 204. The second second-stage wire 106 is hooked around the same form elements 100 as the first second-stage wire 106. The second end of the second second-stage wire is led out to a fourth bobbin 204d of the third arrangement 204. The fourth bobbin 202d is preferably the fourth bobbin 202d of a different group 206 of bobbins than the third bobbin 204c whereto the first end of the second second-stage wire 106 is connected. The different groups 206 of bobbins for forming the group of second-stage apices may be groups 206 adjacent to one another.

The first and second-stage wires may form a first group of second-stage apices. By hooking another pair of second-stage wires 106 around another two form elements 100 a second group of second-stage apices are formed. It is preferred that each two adjacent form elements 100 have a group of second-stage wires 106 hooked around them. Meaning, that thefirst group of second-stage apices is formed around a first and a second form element 100. The second group of second-stage apices may be hooked around a third and a fourth form element. The third group of second-stage apices may thus be formed around the second and third form element 100. Hence, the third group of second-stage apices may envelop the first and second groups of second-stage wires.

Each group of second-stage apices may be located between two adjacent groups of second-stage apices.

In the shown arrangement, the first-stage apices are enveloped by the second-stage apices.

Each group of second-stage apices may be located between two adjacent groups of first-stage apices. Then the braiding process can begin until the tubular base body is formed by braiding. That means after the set-up of connecting the first-stage wires 104 and the second-stage wires 106 to the braiding machine, the machine can braid a tubular base body. During braiding, the first-stage wires 104 may form a first-stage mesh structure. The second-stage wires 106 may form a second-stage mesh structure. The tubular base body may have an opening. The opening may have the shape of the arrangement of the form elements 100. The opening may thus be provided at an end of the base body, here in the example the distal end of the base body. The opening may then be part of a through channel 410 of a medical device 400 as elucidated below.

Advantageously, a sturdy opening is thus formed by the braiding set up at one end of the braided tubular base body.

The opening of some embodiments can thus be provided at an end of the base body without any elements extending beyond the end. Medical devices are thus providable, by forming from the base body, with an opening at an end thereof, without protruding elements (like bundles of wire, securing means, or the like). Such openings provided from base bodies manufactured as described herein provide long-term openings at an end (or a channel through the) medical device thus formed from the base body (preferably by using a mold and heat setting process). The opening does thus advantageously not grow over or cause endothelization over the opening when implanted. Moreover, the base body thus formed provides a sturdy guide at the perimeter of the opening at its end for a forming element inserted into the braiding of the base body, such as a peg, e.g. when inserted into a mold to form a medical device. Thus, the obtained tubular braided base body provides advantageously facilitating to manufacture of a medical implantable device (such as a shunting device or occlusion device/occluder) in a secure and reliable manner.

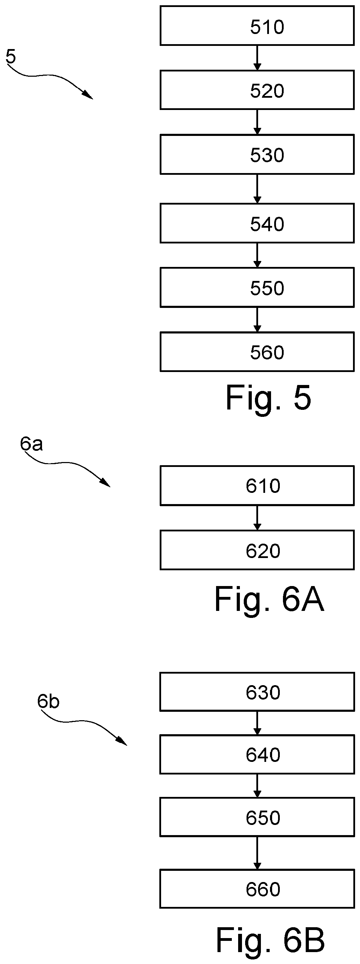

In summary, as shown in Fig. 5, a method 5 is provided in some embodiments for forming a braided tubular base body that preferably has an opening at an end thereof. The base body is suitable for shaping a medical implant. The example of method 5 includes step 510 of providing a plurality of form elements 100 at one end of a braiding head 102. The form elements 102 are provided as disposed of in a first arrangement at the top of the braiding head 102. When the braiding head has a central axis CA, the form elements are provided as disposed of in a first arrangement around the central axis CA. Further, method 5 includes step 520 of providing a plurality of pairs of first-stage wires 104. Method 5 includes step 530 of forming a plurality of groups of first-stage apices by hooking each pair of the first-stage wires 104 over a first and a second form element. Method 5 includes step 540 of providing a plurality of pairs of second-stage wires 106. Method 5 includes step 550 of forming a plurality of groups of second-stage apices each located between adjacent first-stage apices by hooking each pair of second-stage wires 106 over the first and second form elements 100 enveloping the first-stage apices. Method 5 includes step 560 of subsequently braiding the first 104 and second-stage wires 106 over the braiding head 102 starting from the form elements 100 at the top of the tubular base body.

In some preferred embodiments, the method 5 includes a further step of providing a plurality of bobbins 202, 204 arranged in a second and third concentric arrangements outwards from the central axis CA of the braiding head 102, and forming the group of first-stage apices by a first and a second first-stage wire, Ends of the first first-stage wires are led out from the first apex to a first bobbin of the second arrangement and a first bobbin of the third arrangement respectively, wherein the first bobbins of the second and third arrangements are concentrically aligned, Ends of the second first-stage wires are led out from the apex to a second bobbin of the second arrangement and a second bobbin of the third arrangement respectively, the second bobbins of the second and third arrangements being concentrically aligned, The second bobbin of the second arrangement is adjacent the first bobbin of the second arrangement, and the second bobbin of the third arrangement is adjacent the first bobbin of the third arrangement.

Preferably, each pair of first and second bobbins are adjacent to a pair of third and fourth bobbins without any first-stage wires connected thereto.

In some preferred embodiments, the method 5 includes additionally or alternatively a further step of forming the group of second-stage apices are formed by a first and a second second-stage wire, wherein one end of the first second-stage wire is led out to a third bobbin of the second arrangement being adjacent the second bobbin of the second arrangement, while the other end is led out to a fourth bobbin of the third arrangement. One end of the second apex second-stage wire is led out to a third bobbin of the third arrangement being adjacent to the second bobbin of the third arrangement, the other end is led out to a fourth bobbin of the second arrangement, where the fourth bobbins are adjacent a first bobbin of another group of first-stage apices.

In some preferred embodiments, method 5 includes additionally or alternatively that the step of providing a plurality of bobbins comprises providing a ratio of form elements 100 to bobbins 202, 204 is 1:4.

In some preferred embodiments, method 5 includes additionally or alternatively that the step providing a plurality of bobbins 202, 204 comprises providing forty bobbins 202 on the second arrangement, and forty bobbins 204 in the third arrangement.

In some preferred embodiments, method 5 includes additionally or alternatively that the step of providing a plurality of form elements 100 comprises providing ten form elements 100.

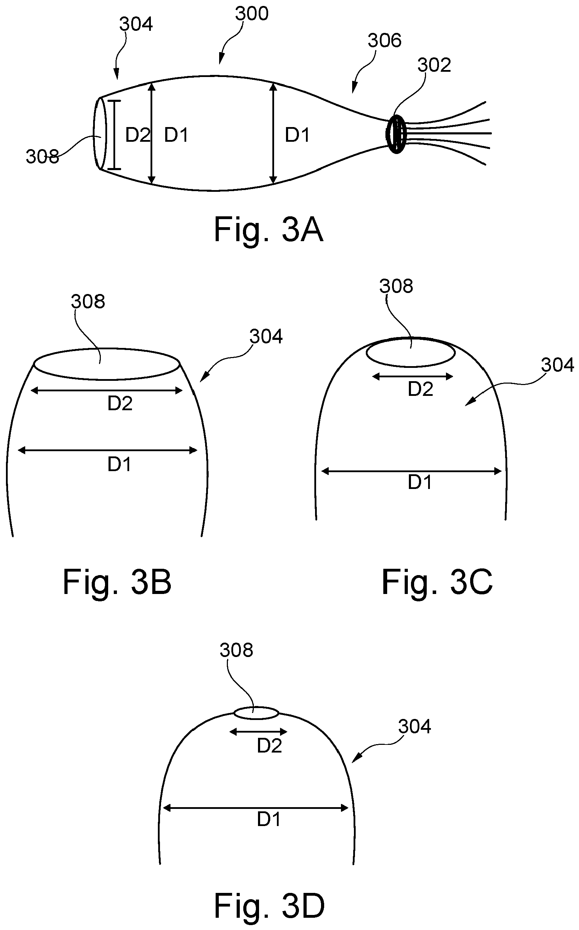

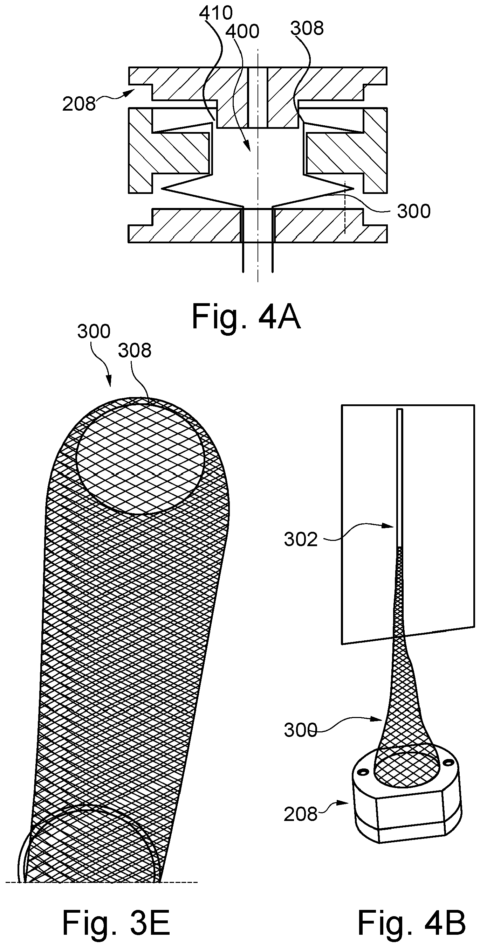

A schematic view of a braided tubular base body is shown in Figure 3A. Another example is shown in Fig. 3E. The braided tubular base body 300 has a distal end portion 304 and a proximal end portion 306. The braided tubular base body 300 may have a cylindrical shape. The cylindrical shape may be circular (in cross-section of the tubular body) cylindrical. The braided tubular base body 300 may thus have a hollow-shaped mesh structure.

The tubular base body 300 has in certain embodiments a cross-sectional diameter D1 at the base of the tubular body, which preferably is cylindrical, as generally determined by the shape of the braiding head 102 body. The opening 308 at an end of the tubular base body 300 may have an opening diameter D2. The cross-sectional diameter D1 of the tubular base body 300 is generally in preferred embodiments wider than the opening diameter D2. In certain embodiments, D2 may be identical to D1 (a straight tube shape). In certain embodiments, D2 may even be larger than D1 (a funnel shape).

The braided tubular base body 300 comprises an opening 308 at the distal end 304. The opening has a perimeter of defined shape. The perimeter shape of the opening may have a circular shape in certain embodiments. It may have an oval shape in some embodiments.

At the proximal end, 306 of the braided tubular base body 300, the free ends of the wires of the braiding extend freely. These ends may be gathered in one or more fixtures 302. A fixture can be removable or permanently arranged. Commonly, a fixture is e.g. a wire wound removably around the bundle of wires not braided before heat setting. It may be removed after heat setting when the risk of unraveling is reduced. The fixture prevents unraveling or fraying of the end of the braided tubular base body 300 opposite the opening.

Another type of fixture may be a clamp, a weld, a bolt, or any other suitable fixture for holding together the free ends of the wires. Permanent fixtures are generally applied after heat setting the braided tubular base body 300 to a medical device as described below. In some examples, the ends of the wires may be braided inward into the braided tubular base body hollow to avoid free ends protruding from the base body. The fixture 302 may preferably be formed to matingly connect with a delivery device. In some examples, the fixture is magnetically connectable to a delivery device. In some examples, the fixture comprises a clasp whereon the delivery device may clasp the medical device.

Figures 3b-3d illustrate schematic views of some examples of openings 308 that may be formed at the distal end 304 of the braided tubular base body 300.

The shape of the opening depends generally on how the form elements 100 are arranged in the braiding machine. Hence, a variety of forms and shapes of the perimeter of the opening are possible by changing the arrangement of the form elements 100. In cases where the form elements 100 are arranged in an oval shape, the opening 308 of the braiding will be oval. Likewise, the size of the opening 308 is dependent on the arrangement of the form elements 100 and their placement on the braiding head 102 in relation to the central axis CA thereof.

The braiding head 102 may have a rounded shape, which when the form elements 100 are arranged inside of the rounded portion forms a cupped form of the distal end of the tubular base body. Hence, the opening may have a smaller diameter than a cross-section of the braided tubular base body 300.

The embodiment shown in Figure 3B has an opening diameter D2 that is almost the same size as the cross-sectional diameter D1 of the braided tubular base body. Such a configuration may be suitable when making for example an occlusion device or shunting device wherein the braiding is inverted. Meaning that the opening of the braiding may be folded outwards when inserted into a mold for forming the medical device.

The embodiment shown in Figure 3C has an opening diameter D2 that is smaller than the cross-sectional diameter D1 of the braided tubular base body. Such a configuration may be beneficial when making a medical device comprising a through the channel. The opening may be used as a guide for any channel formation device (e.g. a peg described below) through the medical device.

The embodiment shown in Figure 3D has an opening diameter D2 that is very narrow compared to the cross-sectional diameter D1 of the braided tubular base body. A small opening that is merely an opening, may be beneficial in order to make an occlusion device having a substantially smooth surface. The braiding may be set up such that the opening is so narrow that it does not compromise the occlusion capabilities of a final medical device.

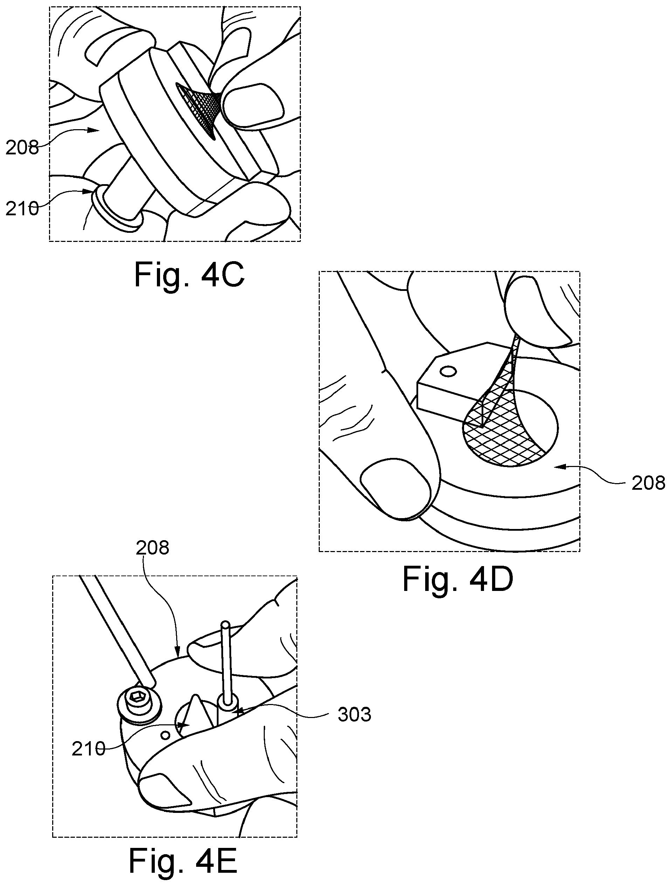

Turning now to Figure 4A, a schematic cross-section view of a mold structure 208 is shown. It should be noted that this is only an example of a mold structure, and that the mold structure used to make a medical device from the braided base body may have different features than disclosed in the figures.

For forming the medical device, the braided tubular base body 300 is inserted in the mold structure 208.

The braided tubular base body 300 may be inserted into the mold structure 208 so that the distal end portion 304 may be folded inwards to create a double layer of braided mesh.

In another example, the distal end portion 304 may be folded outwards when inserted into the mold structure 108. When folding the distal end outwards from the center channel, an inverted braiding structure may be formed. Hence, a medical implantable device may be formed having an inverted braiding.

Figure 4B shows a tubular braided base body 300 being inserted into a mold structure 208. All ends of the wires extend at one side of the mold structure 208. This is a stage before adding the part of the mold structure that forces/decides where the fixing element 302 of the device will be located after heat setting in relation to a central axis of the device. The mold structure 208 may comprise a portion allowing the fixing element 320 at a location offset to the central axis of the medical device. The mold structure 208 may comprise a portion allowing the fixing element 320 at a location along the central axis of the medical device.

As is shown in figure 4C the mold structure 208 may comprise a peg 210. The peg 210 may be inserted into the mold structure 208 for forming a channel through the medical implant. The peg 210 may have a conical tip. The peg 210 may be inserted at the center of the mold structure 208 for forming the channel through a center of the medical implant. Thus, medical devices with improved through channels are advantageously providable.

Figured 4D-4E discloses a closing of a mold structure 208. The ends of the wires are pushed to a side of the mold structure 208 by the peg 210.

The mold structure 208 may have an opening 303 at a distance off-set from the center of the mold structure through where ends of the first-stage wires 104 and/or second-stage wires 106 are protruding from the mold structure 208. The ends of the wires may prior to heat setting the tubular base body be fixated when inserted into the mold. A portion of the ends protruding from end 303 may be fixated together at this opening, see Fig. 4E.

Upon insertion of the braided tubular base body 300 into the mold structure 208, the mold structure 208 and the braided tubular base body 300 is preferably treated with a heat setting. The heat setting forms the shape of the medical implantable device.

The ends of the wires may after heat setting the tubular base body inserted into the mold be fixated. For instance the bundle is welded together to form a spherical fixating element 302. In some examples a clamp may be used when fixating a portion of the ends of the wires together. Such clot welding method is disclosed in international PCT publication WO2009/016265A2 of the same applicant, which hereby is incorporated by reference in its entirety.

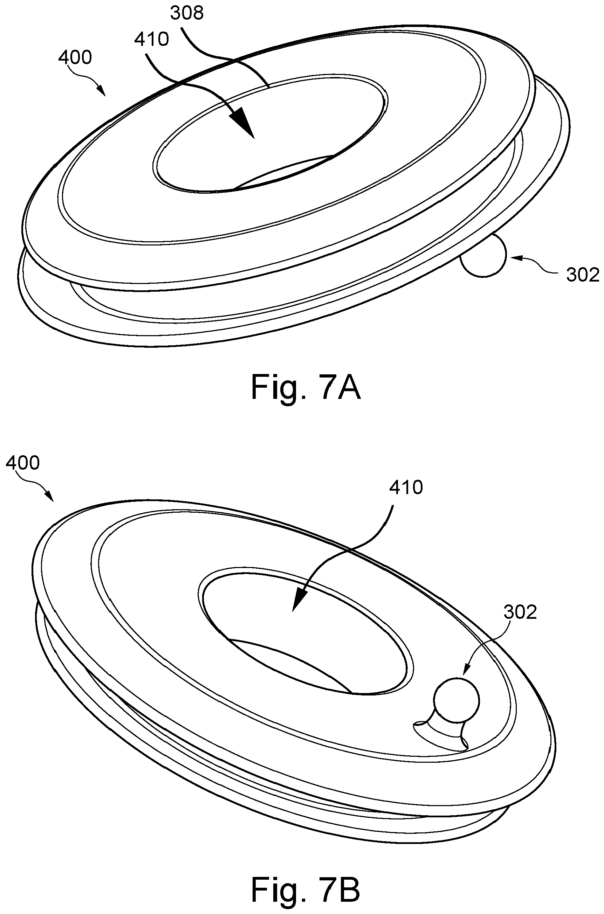

Figures 7A and 7B are schematic illustrations of example of a medical implantable device 400 with a through-channel made in this manner (methods 6a/6b) of a braided tubular base body 300 (made by method 5).

In summary, as shown in Fig. 6A, a method 6a is provided in some embodiments for forming a medical implantable device. The method 6a preferably includes in some examples the step 610 of forming a braided tubular base body 102, as described in the examples above. Method 6a includes a step 620 of heat treating the tubular base body to form the medical implantable device from the braided tubular base body. Heat treatment is preferably made based on a mold as described above. Thus, a medical device, such as the device 400 is manufactured by method 6a.

In further summary of the disclosure, as shown in Fig. 6B, a method 6b is provided for forming a medical implantable device, preferably with a through channel 410. Method 6b includes a step 630 of braiding a tubular base body 102, like step 610 of method 6a, and in some examples as described in the examples above. The tubular body 102 comprises a plurality of groups of first-stage apices and a plurality of groups of second-stage apices each second-stage apex located between adjacent first-stage apices.

Method 6b includes a step 640 of providing a mold structure 208 and inserting the braided tubular base body 102 into the mold structure 208 for shaping a medical device of the tubular base body 102. Method 6b includes a step 650 of inserting a peg 210 through the braided tubular base body 102 inserted in the mold structure 208 for forming a channel through the braided tubular base body 102 and across the mold structure 208.

Method 6b includes a step 650 of heat setting the mold structure 208 with the braided tubular base body 102 and peg 210 to form the medical implantable device 400, like the step 620 of method 6a.

In some preferred embodiments, the method 6b includes additionally or alternatively a further step of inserting the peg at the center of the mold, wherein the peg preferably has a conical tip.

In some preferred embodiments, method 6b includes additionally or alternatively a further step of fixating at least a portion of ends of wires of the braided tubular base body together.

In some preferred embodiments, method 6b includes additionally or alternatively a further step of fixating the wires at a distance off-set a central axis of the medical implantable device.

The medical implant may be an occlusion device. In some examples, a membrane may be added to the medical implant to improve occlusion at selected portions thereof. The medical implant may be a shunting device, such as an atrial flow regulator described in WO 2016/038115A1 of the same applicant, which hereby is incorporated by reference in its entirety (with improved properties provided by the present tubular base body technology disclosed in the present application. A membrane may be affixed over the opening and/or at the through the channel. Thus, an occlusion device is provided that can transform into a shunting device. The membrane may be degradable. After a certain desired implantation time, the membrane will dissolve in the body and provide a throughchannel. Alternatively, the membrane may be nondegradable and pierced in a surgical procedure. The membrane may be controllably removed from the outside of the body. For instance, ultrasonic, radiofrequency, or similar radiation may lead to the disintegration of the membrane or trigger a degradation process. The membrane may be dissolved, avoiding debris in the blood flow. The membrane may be re-closed, e.g. by a stapler or the like. Alternatively, the through channel 410 may include a valve unit. The valve unit may be inserted into through channel 410. The valve unit may in certain examples be an integral part of the medical device 400. The valve unit may in certain examples be inserted and/or affixed to through channel 410 of the medical device 400. The valve unit may in certain examples be a so called stent valve expanded or expandable into the through channel 410. The valve unit may provide for uni-directional flow, e.g., by leaflets in the through-channel. The valve unit may have two operational states (closed/open), operated and selected e.g. by external magnetic triggering or a bistable mechanism. The valve may open or close after a certain time of implantation, e.-g. by a biodegradable locking unit holding the valve in a certain position.

Thus, advantageous therapeutic procedures may be provided for the treatment of patients, avoiding invasive access to the medical device after being implanted.

The medical implant may be an anchoring structure for an improved anchoring of a different medical implantable device. The opening perimeter may provide for advantageous attachment of other structures to the medical device when implanted.

The first-stage wires 104 may have the same thickness as the second-stage wires 106. The first-stage wires 104 may have a different thickness than the second-stage wires 106. The first-stage wires 104 may be thinner than the second-stage wires 106. The first-stage wires 104 may be thicker than the second-stage wires 106. This may provide for the advantageous mechanical strength of the medical device formed from such a base body wire thickness configuration.

The first-stage wires 104 may be made of a shape memory material, such as a shape memory metal alloy. The first-stage wires 104 may be made of a biodegradable material. The first-stage wires 104 may be made of a bioabsorbable material. The first-stage wires 104 may be formed from magnesium. The first-stage wires 104 may be made form a nickel-titanium alloy. This may provide for advantageous desired degradation properties of the medical device formed from such a selected base body wire material.

The second-stage wires 106 may be made of a shape memory material. The second-stage wires 106 may be made of a biodegradable material. The second-stage wires 106 may be made of a bioabsorbable material. The second-stage wires 106 may be formed from magnesium. The second-stage wires 106 may be made form a nickel-titanium alloy. This may provide for advantageous desired degradation properties of the medical device formed from such a selected base body wire material. The first-stage wires 104 and/or the second-stage wires 106 may in some embodiments be made from any other metal or elastic material suitable for the human body. The first-stage wires 104 and the second-stage wires 106 may be made from the same material or different materials.

The present invention has been described above with reference to specific embodiments. However, other embodiments than the above described are equally possible within the scope of the invention. Different method steps than those described above, performing the method by hardware or software, may be provided within the scope of the invention. The different features and steps of the invention may be combined in other combinations than those described. The scope of the invention is only limited by the appended patent claims.

Although modifications and changes may be suggested by those skilled in the art, it is the intention of the inventors to embody within the patent warranted heron all changes and modifications as reasonably and properly come within the scope of their contribution to the art.

### LIST OF REFERENCE NUMERALS IN FIGURES

100 - form element
102 - braiding head
CA - central axis of braiding head
104 - first-stage wire
106 - second-stage wire
202 - bobbins in second arrangement
202a - first bobbin of second arrangement
202b - second bobbin of second arrangement
202c - third bobbin of second arrangement
202d- fourth bobbin of second arrangement
204 - bobbins in third arrangement
204a - first bobbin of third arrangement
204b- second bobbin of third arrangement
204c- third bobbin of third arrangement
204d- fourth bobbin of third arrangement
206 -group of bobbins
208 - mold structure
210 - peg
222 - pair of bobbins
300- braided tubular base body
302- fixing element
303- opening offset center
304- distal end portion of braided body
306- proximal end portion of braided body
308 - opening of tubular base body
400 - implantable medical device
410 - through channel
5 - method
510- 560 method steps
6a - method
610- 620 method steps
6b - method
630- 660 method steps
D1- cross sectional diameter of raided tubular base body
D2- diameter of opening

## Claims

1. A method of forming a braided tubular base body having an opening at an end thereof for a medical implant, said method including:
providing a plurality of form elements (100) at one end of a braiding head (102), said braiding head having a central axis (CA), and said form elements being disposed of in a first arrangement around said central axis (CA),
providing a plurality of pairs of first-stage wires (104);
forming a plurality of groups of first-stage apices by
hooking each pair of said first-stage wires (104) over a first and a second form element;
providing a plurality of pairs of second-stage wires (106);
forming a plurality of groups of second-stage apices each located between adjacent first-stage apices by hooking each pair of second-stage wires (106) over said first and second form elements (100) enveloping said first-stage apices
and subsequently braiding said first (104) and second-stage wires (106) to said tubular base body.

2. The method of claim 1 further comprising:
providing a plurality of bobbins (202, 204) arranged in second and third concentric arrangements outwards from said central axis (CA) of said braiding head (102) and wherein said group of first-stage apices is formed by a first and a second first-stage wire,
wherein ends of said first first-stage wires are led out from the first apex to a first bobbin of the second arrangement and a first bobbin of the third arrangement respectively, wherein said first bobbins of the second and third arrangements being concentrically aligned,
wherein ends of said second first-stage wires are led out from the apex to a second bobbin of the second arrangement and a second bobbin of the third arrangement respectively, said second bobbins of the second and third arrangements being concentrically aligned,
wherein said second bobbin of the second arrangement is adjacent said first bobbin of the second arrangement, and
wherein said second bobbin of the third arrangement is adjacent said first bobbin of the third arrangement.

3. The method of claim 2, wherein each pair of first and second bobbins are adjacent to a pair of third and fourth bobbins without any first-stage wires connected thereto.

4. The method of claim 2 or 3, wherein said group of second-stage apices are formed by a first and a second second-stage wire, one end of said first second-stage wire is led out to a third bobbin of the second arrangement being adjacent to the second bobbin of the second arrangement, the other end is led out to a fourth bobbin of the third arrangement, and
one end of said second apex second-stage wire is led out to a third bobbin of the third arrangement being adjacent the second bobbin of the third arrangement, the other end is led out to a fourth bobbin of the second arrangement,
said fourth bobbins being adjacent to afirst bobbin of another group of first-stage apices.

5. The method of any one of claims 2-4, wherein providing a plurality of bobbins comprises providing a ratio of form elements (100) to bobbins (202, 204) being 1:4.

6. The method of any of claims 2-5, wherein providing a plurality of bobbins (202, 204) comprises providing forty bobbins (202) on said second arrangement, and forty bobbins (204) in said third arrangement.

7. The method of any one of the preceding claims, wherein providing a plurality of form elements (100) comprises providing ten form elements (100).

8. A method for forming a medical implantable device, comprising:
forming a braided tubular base body according to any one of claims 1-7; and
heat treating the tubular base body to form said medical implantable device from said braided tubular base body.

9. A method of forming a medical implantable device with a through channel, the method comprising:
braiding a tubular base body, preferably according to the method any one of claims 1-7, wherein said tubular body comprises a plurality of groups of first-stage apices and a plurality of groups of second-stage apices each second-stage apex located between adjacent first-stage apices;
providing a mold structure and inserting the braided tubular base body into said mold structure;
inserting a peg through said braided tubular base body inserted in said mold structure for forming a channel;
heat setting said mold structure with said braided tubular base body and peg to form said medical implantable device.

10. The method of claim 9 wherein said peg is inserted at a center of the mold, wherein said peg preferably has a conical tip.

11. The method of claim 9 or 10 further fixating at least a portion of ends of wires of said braided tubular base body together.

12. The method of claim 11, comprising fixating said wires in at a distance offset a central axis of said medical implantable device.

13. A braided tubular base body (300) for a medical implant, preferably a shunting or occlusion device, said base body comprising an opening at an end thereof, wherein a perimeter of said opening is formed of:
a plurality of first-stage wires, and a plurality of second-stage wires
a plurality of groups of first-stage apices; and
a plurality of groups of second-stage apices each located between adjacent first-stage apices, wherein said base body preferably is manufactured in accordance with the method any of claims 1-7.

14. An implantable medical device (400), preferably made from a tubular base body according to claim 13 and/or manufactured in accordance with the method of claim 8 or 9, said medical implantable device comprising at an end thereof and opening with a perimeter at which is located:
a plurality of first-stage wires, and a plurality of second-stage wires
a plurality of groups of first-stage apices; and
a plurality of groups of second-stage apices each located between two adjacent first-stage apices.

15. The implantable medical device according to claim 14, wherein said medical implantable device comprises a through-channel (410) through said medical implantable device including said opening.
